# EUROPEAN PATENT SPECIFICATION

(11) **EP 1 011 563 B1**
(45) Date of publication and mention of the grant of the patent: **09.11.2005**
(21) Application number: 98922772.3
(22) Date of filing: 24.04.1998
(51) Int. Cl.: A61F 9/007

(54) **DUAL MODE OPHTHALMIC LASER ABLATION**
DUAL-MODE OPHTHALMISCHE LASERABLATION
ABLATION LASER OPHTALMIQUE A DOUBLE MODE

(30) Priority: 25.04.1997 US 44908 P
(43) Date of publication of application: 28.06.2000
(73) Proprietor: Technolas GmbH Ophthalmologische Systeme, 85622 Feldkirchen (DE)
(72) Inventor: HOHLA, Kristian, D-85591 Vaterstetten (DE)
(74) Representative: Vossius & Partner
(86) International application number: PCT/EP1998/002428
(87) International publication number: WO 1998/048746

(56) References cited:
- EP-A- 0 412 789
- WO-A-92/16141
- WO-A-94/07447
- WO-A-96/11655
- WO-A-96/21407
- WO-A-97/46183
- DE-A- 4 232 915
- DE-C- 4 103 493

## Description

The invention relates to an apparatus and technique for surgically modifying the curvature of the eye cornea and a method of controlling the apparatus, and more particularly to an apparatus for immediately correcting a variety of corneal defects using dual, fixed spot sizes.

Excimer laser eye surgery systems are often used for correcting vision. From eye glasses to radial keratotomy, ophthalmic surgery has now progressed to a point where the surface of the eye is actually reshaped using cold light laser ablation provided by excimer lasers, typically argon fluoride lasers operating at around 193 nanometers. These lasers are even used to reshape the stromal tissue underneath the surface of the eye in a laser *in situ* keratomileusis technique patented by Gholam Peyman in U.S. Patent No. 4,840,175.

These techniques start with the uncorrected profile of the eye, and then ablate the eye using various small or large beam techniques, or aperture techniques, to reprofile the surface into a desired, corrected profile. The amount of correction is determined by a variety of methods, but for myopia, for example, given the starting curvature of the eye and the amount of dioptric correction needed, equations are well known which specify the amount of tissue that must be removed from each point on the surface of the eye. These equations are found, for example, in PCT Patent Application Serial No. PCT/EP93/02667. Similar equations are known for the amount of tissue necessary for removal to correct for hyperopia and astigmatism.

Before relying on these equations, however, the actual curvature of the eye must be determined. This is done using a number of techniques. The patient's visual acuity can be determined through eye exams. The actual shape of the surface of the eye can be determined, for example, using a topography system. These topography systems can be either manual or computerized, and the latter can provide a point-by-point representation of the curvature of the eye, for example, in the form of an axial curvature, the instantaneous or true local curvature, or the absolute height.

Typically, based on these curvatures and a patient's visual acuity, a doctor programs into an excimer laser surgery system an amount of positive or negative dioptric correction (depending on whether the correction is for hyperopia or myopia) and an angle of the cylinder of astigmatism, if any, along with the amount of dioptric correction necessary for the astigmatism. Software within the excimer system itself then calculates a shot pattern suitable for achieving the objective, and that pattern is executed on the surface of the patient's eye.

International Publication No. WO 97/46183 (International Application No. PCT/EP97/02721) discloses a distributed system for controlling excimer laser eye surgery. A topography system, a computer system, and an excimer laser eye surgery system are provided, with the topography system providing profile data to the computer system, and the computer system calculating and providing an ablation shot pattern to the excimer laser eye surgery system. The computer system and the excimer laser eye surgery system can be located remotely. The excimer laser eye surgery system can receive data from more than one computer system and from more than one topography system for better utilization of resources.

A number of systems have been developed to reshape the cornea, using a variety of techniques such as variable sized circular apertures to correct for myopia, variable sized ring shaped apertures to correct for hyperopia, and variable sized slit shaped apertures to correct for astigmatism. These techniques collectively came to be known as photorefractive keratectomy. It has been recognized that using such apertures to correct for myopia, for example, a series of excimer laser shots using progressively smaller spot sizes could ablate away a portion of the cornea to effectively build a "corrective lens" into the cornea.

These techniques are discussed, for example, in U.S. Patent No. 4,973,330, entitled "Surgical Apparatus for Modifying the Curvature of the Eye Cornea," issued November 27, 1990, and in U.S. Patent No. 4,729,372, entitled "Apparatus for Performing Ophthalmic Laser Surgery," issued March 8, 1988. Those skilled in the art of laser ophthalmological surgery have extensively developed the required exposure patterns using these variable size apertures to provide an appropriate amount of correction to various degrees of myopia, hyperopia, and astigmatism, and a combination of these conditions. These multiple aperture systems tend to be complicated and inflexible. A number of aperture wheels or masks are required, and only standard forms of correction for myopia and hyperopia with circular symmetry and astigmatism with cylindrical symmetry are provided.

An apparatus for ablating tissue from the eye is shown in U.S. Patent No. 4,973,330, referenced above. This apparatus includes an excimer laser, the laser beam of which impinges on the cornea, with the axis of the laser beam coinciding with the optical axis of the eye. Furthermore, a field stop limits the area of the laser spot on the cornea illuminated by the laser beam, and the size of this field stop is set in a temporarily variable manner according to the profile of the area to be removed so that the thickness of the area to be removed is a function of the distance from the optical axis of the eye.

The system described in U.S. Patent No. 4,973,330 permits in this way setting the "laser energy deposited" on the cornea as the function of the distance from the optical axis of the eye, but only under the condition that the distribution of energy (i.e., the power of the laser beam spot) is homogeneous, or at least axially symmetrical. This, however, is a condition that excimer lasers in particular do not always fulfill. Inhomogeneous power distribution results in non-axially symmetrical removal. Moreover, the system described in U.S. Patent No. 4,973,330 only permits the correction of spherical aberrations, not astigmatism.

An apparatus based on the same fundamental idea is known from U. S. Patent No. 4,994,058, entitled "Surface Shaping Using Lasers", issued February 19, 1991. That apparatus employs a "destructible field stop mask" instead of a field stop having a temporarily variable aperture.

Another class of apparatus for shaping the cornea by means of removing tissue is known from the various L'Esperance patents. These include U.S. Patent Nos. 4,665,913; 4,669,466; 4,718,418; 4,721,379; 4,729,372; 4,732,148; 4,770,172; 4,773,414; and 4,798,204. In that apparatus, a laser beam with a small focus spot is moved by a two-dimensional scanning system over the area to be removed. This apparatus, which operates as a "scanner," has the advantage that it can generate any two-dimensional profile of deposited energy "over the area to be removed."

International Publication No. WO 96/11655 (International Application No. PCT/EP95/04028) discloses an apparatus and method for controlling an apparatus for removing tissue from the eye, performing various types of corrections using a relatively large beam, but oscillating or dithering to prevent reinforcing ridges from being formed during the tissue removal process. Various types of correction, such as hyperopia and astigmatism correction, are performed using a large beam that is scanned over the area to be ablated using overlapping shots.

Using an infrared fluorescent dye to dye the epithelium, the epithelium in the area to be treated is removed while observing the fluorescent patterns from the epithelium. Once a certain area is no longer fluorescent after laser shots, smaller shots are then applied, selectively removing the epithelium from the remaining regions. Using two astigmatism correcting ablation patterns intersecting at an angle, a lens is created capable of correcting for myopia, hyperopia, and astigmatism. Overlapping shots, using a relatively large fixed spot size, provide for reduced thermal heating, ridgeless treatment patterns, reduced shot count and simplified equipment. Thus, this reference illustrates a single fixed spot size system using a large fixed spot in an overlapping pattern to correct vision.

With the various advances, excimer laser eye surgery systems implement a variety of techniques to re-profile the surface of the eye. The use of large spot sizes reduces treatment time and increases the amount of tissue removed per shot, but small spot sizes provide for finer resolution of correction. A technique and apparatus with the advantages of both would be greatly desirable.

An apparatus according to the first part of claim 1 is known from WO-A-94/07447.

According to the invention, a dual mode excimer laser eye surgery system is provided. In this system, the eye is first treated for primary corneal defects, such as myopia, hyperopia, and astigmatism, using a large, fixed spot size. Then, a small fixed spot size is used to remove remaining irregularities. The large size allows for faster treatment. The small size provides for more precision in the treatment of irregular topographies.

Further, such a system is preferably implemented in a distributed topography environment. For example, a treatment pattern using the large, fixed spot size is provided to doctors based on visual acuity data, such as the degree of dioptric correction needed. Then, the effect of this treatment is overlaid on a computer against the patient's actual eye topography. The doctor then uses the small fixed spot size to remove any remaining irregularities yielding a preferred treatment pattern. This combined treatment pattern is then distributed to an excimer laser eye surgery system that performs the large spot size ablation and then the small spot size ablation.
Fig. 1 is a simplified diagram illustrating a typical excimer laser eye surgery system.
Fig. 1A illustrates a dual fixed diaphragm that would replace the variable diaphragm in the excimer laser eye surgery system of Fig. 1, wherein a large spot size diaphragm opening is positioned in the path of a laser beam, according to the present invention.
Fig. 1B illustrates the dual fixed diaphragm of Fig. 1A, wherein a small spot size diaphragm opening is positioned in the path of the laser beam, according to the present invention.
Fig. 2 is an illustration of portion of a first pass for correcting vision using the large fixed spot size.
Fig. 2A is an illustration of a typical shot pattern.
Fig. 3 is an illustration of the second pass for correcting vision using a smaller fixed spot size.
Fig. 4 is a block diagram illustrating the interrelationship of multiple components in an excimer laser eye surgery system.

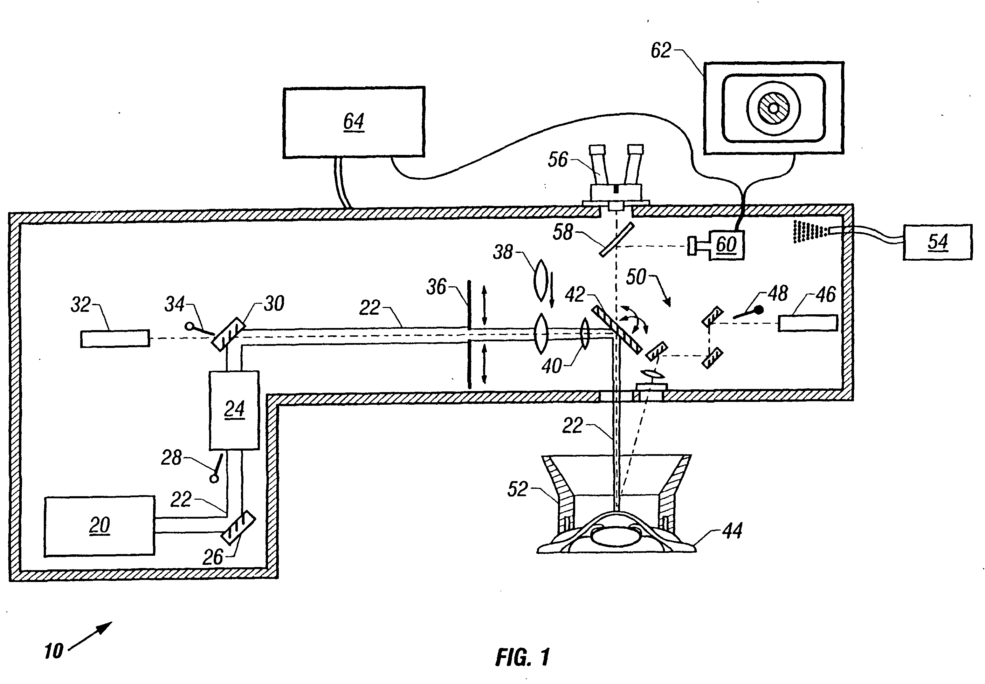

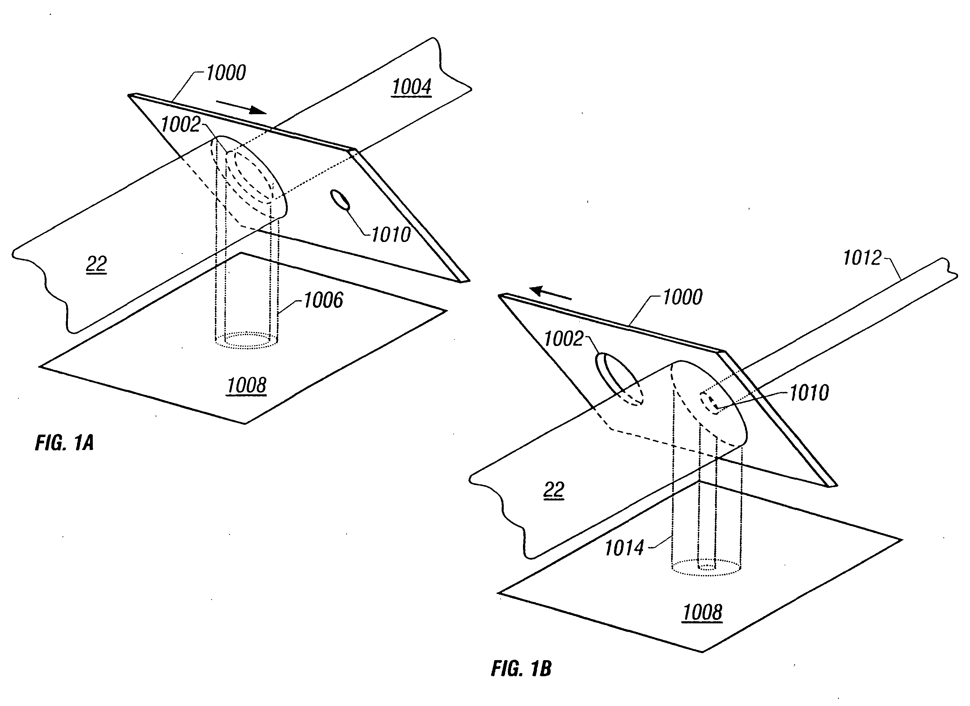

Fig. 1 shows a typical eye surgery system 10. An excimer laser 20 provides a pulsed beam 22 to a beam homogenizer 24 after reflection from optics 26. A shutter 28 is also provided to block transmission of the pulsed beam 22 to the beam homogenizer 24. The excimer laser 20 is a typical excimer laser as is well known in the art. It preferably provides a 193 nm wavelength beam with a maximum pulse energy of 400 mJ/pulse. The excimer laser 20 preferably provides maximum power at the treatment site of 1 W, with a pulse frequency of 10 Hz and a pulse length of 18 ns. By way of example, the wavelength of the light from the laser is preferably less than 400 nm, as that provides the desired ablating action with reduced thermal heating. Further, other pulse energies can be provided, such as all the way down to 200 mJ/pulse, with typical repetition rates of 60 to 100 pulses per second with a typical pulse length of 10 to 30 ns.

The beam homogenizer 24 preferably includes standard homogenization and focusing hardware, which can be based both on optical mixing of the beam and on rotation of the beam. From the beam homogenizer 24, the pulsed beam 22 is then reflected off of optics 30, which also passes a red pilot laser beam from a pilot laser 32. This pilot laser 32 is preferably a 633 nm helium neon laser of less than 1 mW of power. The red pilot beam from the pilot laser 32 can also be blocked by a shutter 34. The pilot laser 32 is aligned so that its optical pathway coincides with the pulsed beam 22. The pilot laser 32 provides the functions of centering the beam 22 on the axis of treatment of the eye 44 and also provides for focusing on the eye 44. Further, it can provide an optical fixation point for the patient, although a different laser or light source could also be provided for that purpose.

From the optics 30, the pulsed beam 20 (now also co-aligned with the beam from the pilot laser 32) then passes through an adjustable diaphragm 36, which allows the beam size to be adjusted before it enters the final optics. After the diaphragm 36, a spot mode lens 38, when in place, provides further concentration of the beam 22, allowing spot ablation of certain defects in the eye by a physician performing therapeutic rather than refractive surgery. The spot mode lens 38 is thus moved into and out of place depending on whether therapeutic or refractive treatment is desired.

Following the spot mode lens 38, a focusing lens 40 directs the beam 22 onto the scanning mirror 42, which then reflects the beam 22 onto a patient's eye 44. Note that the portion of the beam 22 from the pilot laser 32 is used for both adjusting the distance of the eye 44 from the entire eye surgery system 10 and for providing centering, as will be discussed below. The focusing lens 40 focuses light such that when the eye 44 is at the optimal distance, the beam 22 is properly focused onto the eye 44.

These various lenses and mirrors thus combine to form an optical system providing an excimer beam to the cornea. The optical system creates a laser spot on the cornea, and the spot size is adjustable, along with its location. It will be readily appreciated that a wide variety of different systems could be used to optically provide such a beam. For example, a lens could be used to adjust the spot size rather than an aperture, and instead of a scanning mirror, the patient or the patient's eye 44 could be physically moved to provide for shots at different locations on the eye 44.

Also provided in the system is a focusing laser 46, whose beam can also be blocked by a shutter 48. The focusing laser 46 is preferably a green helium neon laser providing a beam of a wavelength of 535 nm and less than 1 mW of power. The beam from the focusing laser 46 travels through optics 50 and impinges on the eye 44 at an angle. The distance of the eye 44 from the eye surgery system 10 is adjusted such that both the beam from the pilot laser 32 and the beam from the focusing laser 46 impinge on the surface of the eye 44 at the same point.

Further provided is an optional fixation mask 52, which is well known in the art and is used to stabilize the eye 44 during surgery. It can include debris removal components, and is typically attached to the eye 44 through either a vacuum suction ring or through hooks. A clean gas purge unit 54 ensures that the optics and the beams in the system are free from any floating debris.

A microscope 56 is provided for the physician to observe progress during ablation of the surface of the eye 44. The microscope 56 is preferably a ZEISS OPMI "PLUS" part No. 3033119910, with magnifications of 3.4, 5.6 and 9.0 times. Field illumination is provided by a cold light source not shown, which is preferably the Schott KL 1500 Electronic, ZEISS part number 417075. This microscope 56 focuses through the scanning mirror 42 and also focuses through a splitting mirror 58. The splitting mirror further provides a view of the eye 44 to an infrared video unit 60, which is used for the epithelial ablation discussed below. The infrared video unit 60 preferably provides an image output to a capturing video screen 62 and to a control unit 64. The infrared video unit 60 is preferably sensitive to both infrared light and visible light.

The control unit 64, which is typically a high performance computer compatible with an IBM PC by International Business Machines Corp., further preferably controls all components of the eye surgery system 10, including the shutters 28, 34, and 48, the diaphragm 36, the spot mode lens 38, and the scanning mirror 42.

With reference to Figs. 1A and 1B, a typical dual diaphragm is illustrated that would be used according to the invention to implement dual fixed spot size excimer laser surgery. According to the invention, for example, one would replace the variable diaphragm 36 (Fig. 1) with the dual fixed diaphragm illustrated in Figs. 1A and 1B. Specifically, a dual diaphragm plate 1000 is slidable to the left or to the right. It is placed in a path of laser beam 22 (Fig. 1) and shifted between its two positions to provide two different spot sizes of the beam. In Fig. 1A, the diaphragm plate 1000 is in a first position in which a large spot sized diaphragm opening 1002 is positioned in the path of the laser beam 22. As is seen, this large diaphragm opening 1002 passes a circular beam 1004 of a first size. A remainder 1006 of the beam 22 is reflected into a laser dump 1008 where it is absorbed.

When it is desired to employ the second, smaller spot size, as illustrated in Fig. 1B, the diaphragm plate 1000 is shifted into a second position. In this position, a smaller diaphragm opening 1010 passes a smaller laser spot 1012. Again, a remainder 1014 of the beam 22 is reflected into the laser dump 1008.

It will be appreciated that the actual size of the spots of the diaphragm openings 1002 and 1010 will not necessarily exactly coincide with the size of a spot that alights on the eye 44. However, it will be appreciated that these two spot sizes will provide for two different sizes of beam impingement on the eye 44. Preferably, the diaphragm opening 1002 is of a size to form an approximately two millimeter diameter spot size on the eye, whereas the opening 1010 is of a size appropriate to form a one millimeter diameter spot size. Other sizes can be used, preferably with the first size large enough to fairly quickly perform a basic ablation pattern, with the second size being relatively small, small enough to provide precise correction of any remaining defects.

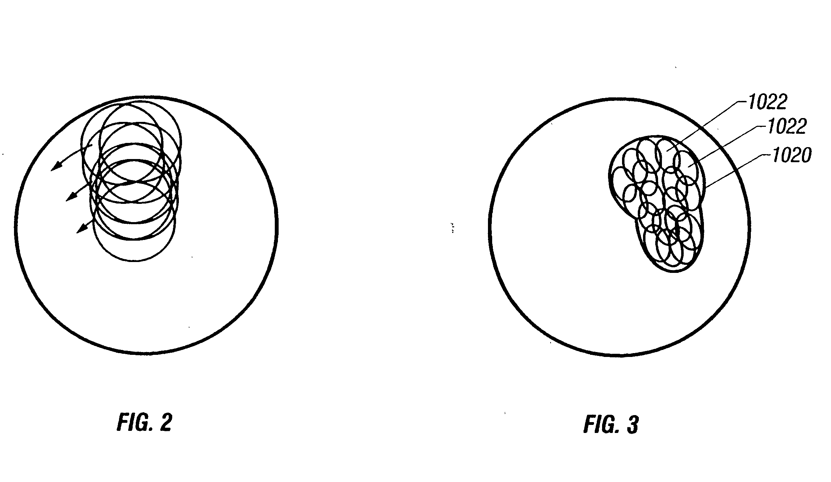

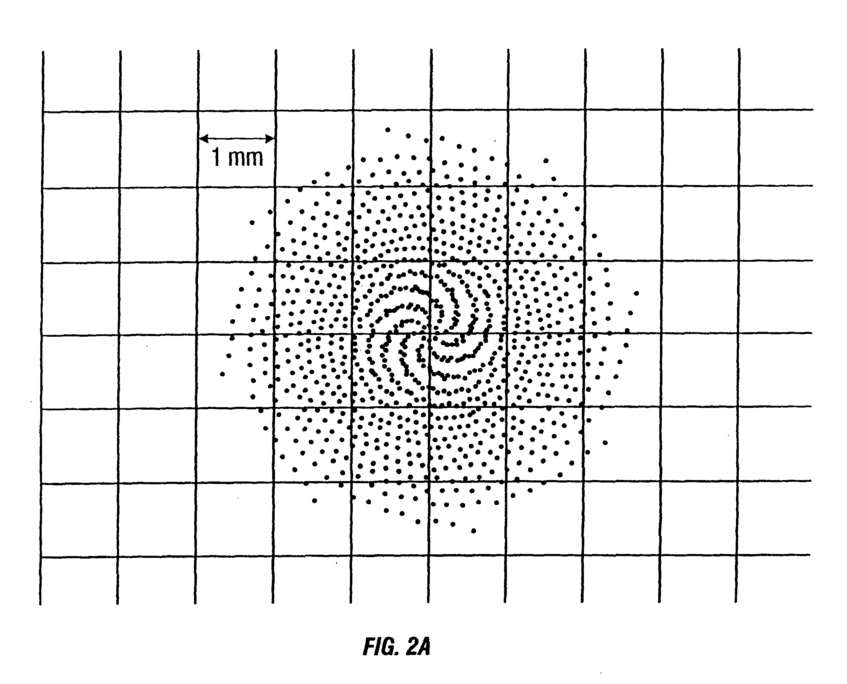

Turning to Figure 2, illustrated is a typical first pass on a 6 millimeter treatment zone using a 2 millimeter spot on the eye formed by the first diaphragm opening 1002. This is illustrative only, and preferably would be a pattern such as that illustrated in Fig. 2A. For additional patterns and methods of operation, see International Publication No. WO 96/11655 (International Application No. PCT/EP95/04028), particularly Figs. 19-28 and the related discussion. Using a large, 2 millimeter spot, a basic ablation profile is achieved.

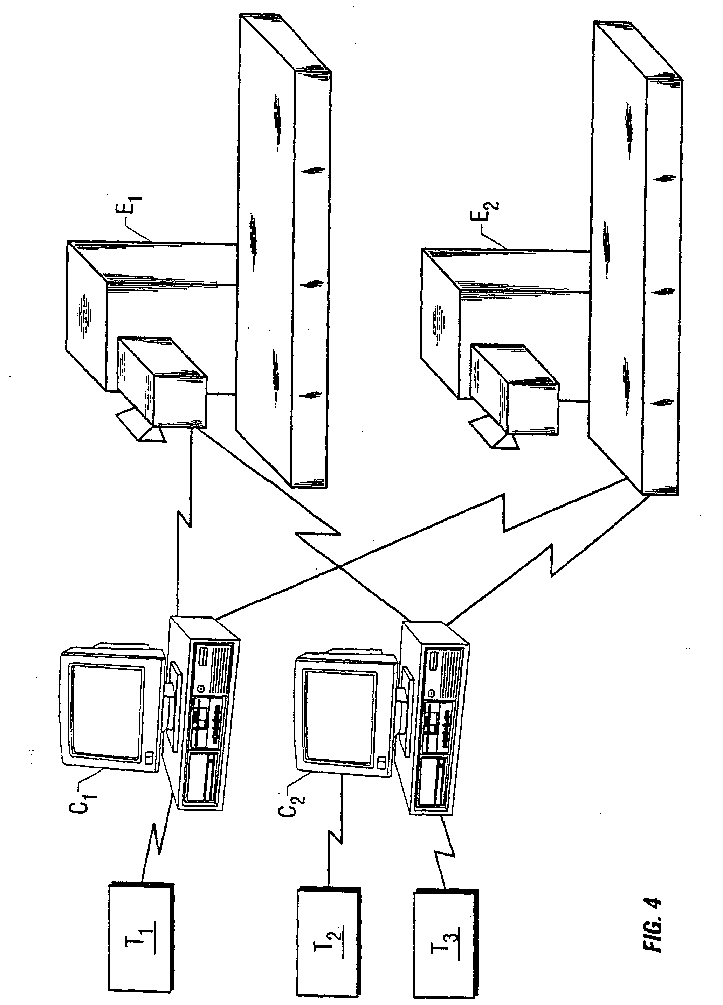

Then turning to Figure 3, assume that after the basic ablation performed in Figure 2, a remaining area 1020 is irregular compared to a desired eye profile. Then, the smaller spot formed by the opening 1010 is used to create a plurality of shots 1022 that "smooth" the remaining irregularity.

Preferably, the dual spot size system according to the invention is used in conjunction with a topography system. With reference to Fig. 4, for example, a topography system T1 is used in conjunction with other visual acuity techniques to determine the degree and type of correction a patient needs, such as for myopia, hyperopia, or astigmatism. A basic treatment pattern is developed by a computer C1 based on the topographic data provided by the topography system T1 and implemented on an excimer laser eye surgery system E1. Second and third topography systems T2 and T3, respectively, are coupled to a computer system C2, which is coupled to an excimer laser eye surgery system E2 and also to eye surgery system E1. Computer C1 is also coupled to eye surgery system E2, and all of these components together provide a distributed topography, treatment creation and excimer laser system. See International Publication No. WO 97/46183 (International Application No. PCT/EP97/02721) for more information on distributed excimer laser surgery systems.

The basic treatment pattern, however, may need refinement because of irregularities in the profile of the patient's eye. Therefore, the doctor could compare the calculated results of the basic treatment pattern using the large spot size with the desired topography. Then, simulating the use of the smaller spot size, the doctor could use free hand techniques to provide appropriate shots within the treatment pattern to correct for the remaining irregularity, resulting, for example, in the treatment of Figure 3. This detailed correction could either be provided with computer assistance suggesting a sequence of smaller shots to fill in the irregular area, or perhaps using a cursor, mouse, or other pointing device to allow the doctor to "paint" in the area to be treated. The computer system would then calculate the theoretical result of ablating an eye with the topography determined by the topography system T1, and hopefully this would be within an acceptable error limit. If not, the doctor could provide further refinements using the smaller spot size.

Therefore, using the techniques according to the invention, a large, fixed spot size is used to provide for basic correction of hyperopia, myopia, or astigmatism. Then, any remaining irregularities are removed using the smaller fixed spot size. Further, the large spot size pattern can be automatically calculated, with the small spot size being manually "painted in" by the physician, or could be automatically calculated under the physician's supervision.

It will be appreciated that a variety of techniques can be used to provide the two spot sizes. For example, instead of using the sliding dual diaphragm, a variable diaphragm as in the diaphragm 36 of Figure 1 could be used, but simply programmed to only assume two different sizes. One skilled in the art will appreciate the variety of techniques to form two discrete sizes.

Preferably, the large fixed spot size uses a large, fixed spot size scanning technique, and the small fixed spot size is provided with small overlapping shots.

The foregoing disclosure and description of the invention are illustrative and explanatory thereof, and various changes in the size, shape, materials, components, circuit elements, and optical components, as well as in the details of the illustrated system and construction and method of operation may be made without departing from the spirit of the invention.

## Claims

1. An apparatus (10) for shaping the cornea by removing tissue from a region of the cornea that has an area to be subject to ablation to a desired treatment pattern, comprising: a laser (20) that emits a laser beam (22) having a suitable wavelength, an optical system (40, 42) coupled to said laser that receives the laser beam and delivers the laser beam onto the region of the cornea, said optical system controllably deflecting the laser beam to different points on the region of the cornea,
**characterized by**
dual spot optics (36, 38) coupled to the optical system and restricting the optical system to provide the laser beam only at a larger, single fixed spot size on the region of the cornea and a smaller, single fixed spot size on the region of the cornea, wherein the larger fixed spot size on the region of the cornea is a relatively large fraction of the area of the region of the cornea to be subject to ablation, and the smaller fixed spot size on the region of the cornea is relatively small compared to the larger fixed spot size; and
a controller (64) for directing the optical system and for directing said laser to fire the laser beam and the optical system to image the laser beam in a series of shots at a plurality of different points on the region of the cornea only at the larger fixed spot size and the smaller fixed spot size, which together cummulatively create the desired treatment pattern.

2. The apparatus of claim 1, wherein the dual spot optics includes a dual fixed diaphragm (36, 1000).

3. The apparatus of claim 2, wherein the dual fixed diaphragm is placed in the path (22) of the laser beam.

4. The apparatus of claim 2, wherein the dual fixed diaphragm slides between a first position and a second position.

5. The apparatus of claim 2, wherein the dual fixed diaphragm has a first opening (1002) and second opening (1010) that is smaller than the first opening.

6. The apparatus of claim 1, wherein the dual spot optics includes a dual fixed diaphragm placed in the path of the laser beam, the dual fixed diaphragm sliding between a first position and a second position and having a first opening and a second opening smaller than the first opening, the laser beam passing through the first opening when the dual fixed diaphragm is in the first position and through the second opening when the dual fixed diaphragm is in the second position.

7. The apparatus of claim 1, wherein the dual spot optics - is a variable diaphragm (36) that is programmably controlled to only assume two different sizes.

8. The apparatus according to any of claims 1 to 7, further **characterized by**
a topography system (T1, T2, T3) that provides profile data corresponding to the profile of the cornea of a patient;
a computer system (C1, C2) with a program for developing an initial ablation shot pattern and a refined shot pattern which together form an overall desired shot pattern from the profile data;
a first data link between said topography system and said computer system for transmission of the profile data from said topography system to said computer system;
and
a second data link between said computer system and said laser eye surgery apparatus for transmission of the initial ablation shot pattern and the refined shot pattern from said computer system to said laser eye surgery apparatus, wherein said laser eye surgery apparatus is located in a physically different vicinity than said computer system.

9. The apparatus of any of claims 1 to 8, wherein the laser is an excimer laser.

10. The apparatus of any of claims 1 to 9, wherein the larger fixed spot size laser beam has a diameter of at least 2.0mm.

11. The apparatus of any of claims 1 to 10, wherein the smaller fixed spot size laser beam has a diameter of not more than 1.0 mm.

12. The apparatus of any of claims 1 to 11, wherein the dual spot optics is a lens.

13. The apparatus of any of claims 1 to 12, wherein the smaller fixed spot size series of shots are overlapping shots.

## Patentansprüche

1. Vorrichtung (10) zur Formung der Cornea durch Entfernung von Gewebe aus einem Bereich der Cornea, der eine Fläche aufweist, die einer Abtragung zu einem erwünschten Behandlungsmuster unterzogen werden soll, mit: einem Laser (20), der einen Laserstrahl (22) emittiert, der eine geeignete Wellenlänge aufweist, einem optischen System (40, 42), das an den Laser gekoppelt ist, das den Laserstrahl aufnimmt und den Laserstrahl auf den Bereich der Cornea abgibt, wobei das optische System steuerbar den Laserstrahl auf unterschiedliche Punkte auf dem Bereich der Cornea ablenkt, **gekennzeichnet durch**
eine Doppelfleckoptik (36, 38), die mit dem optischen System gekoppelt ist und das optische System darauf beschränkt, den Laserstrahl nur mit einer größeren, einzelnen festen Fleckgröße auf den Bereich der Cornea und einer kleineren, einzelnen festen Fleckgröße auf den Bereich der Cornea bereitzustellen, wobei die größere feste Fleckgröße auf dem Bereich der Cornea ein verhältnismäßig großer Bruchteil der Fläche des Bereichs der Cornea ist, die einer Abtragung unterzogen werden soll, und die kleinere feste Fleckgröße auf dem Bereich der Cornea verglichen mit der größeren festen Fleckgröße verhältnismäßig klein ist;.und eine Steuereinrichtung (64) zum Richten des optischen Systems und zum Richten des Lasers, um den Laserstrahl abzufeuern, und des optischen Systems, um den Laserstrahl in einer Reihe von Schüssen auf mehrere unterschiedliche Punkte auf dem Bereich der Cornea nur mit der größeren festen Fleckgröße und der kleineren festen Fleckgröße abzubilden, die zusammen kumuliert das erwünschte Behandlungsmuster erzeugen.

2. Vorrichtung nach Anspruch 1, wobei die Doppelfleckoptik eine doppelte feste Blende (36, 1000) aufweist.

3. Vorrichtung nach Anspruch 2, wobei die doppelte feste Blende im Weg (22) des Laserstrahls angeordnet ist.

4. Vorrichtung nach Anspruch 2, wobei sich die doppelte feste Blende zwischen einer ersten Position und einer zweiten Position verschiebt.

5. Vorrichtung nach Anspruch 2, wobei die doppelte feste Blende eine erste Öffnung (1002) und eine zweite Öffnung (1010) aufweist, die kleiner als die erste Öffnung ist.

6. Vorrichtung nach Anspruch 1, wobei die Doppelfleckoptik eine doppelte feste Blende aufweist, die im Weg des Laserstrahls angeordnet ist, wobei sich die doppelte feste Blende zwischen einer ersten Position und einer zweiten Position verschiebt und eine erste Öffnung und eine zweite Öffnung aufweist, die kleiner als die erste Öffnung ist, wobei der Laserstrahl durch die erste Öffnung geht, wenn sich die doppelte feste Blende in der ersten Position befindet, und durch die zweite Öffnung geht, wenn sich die doppelte feste Blende in der zweiten Position befindet.

7. Vorrichtung nach Anspruch 1, wobei die Doppelfleckoptik aus einer variablen Blende (36) besteht, die programmierbar so gesteuert wird, daß sie nur zwei unterschiedliche Größen annimmt.

8. Vorrichtung nach einem der Ansprüche 1 bis 7, die ferner **gekennzeichnet ist durch**
ein Topographiesystem (T1, T2, T3), das Profildaten liefert, die dem Profil der Cornea eines Patienten entsprechen;
ein Computersystem (C1, C2) mit einem Programm zur Entwicklung eines Anfangsabtragungsschußmusters und eines verfeinerten Schußmusters, die zusammen ein erwünschtes Gesamtschußmuster bilden, aus den Profildaten;
einer ersten Datenverbindung zwischen dem Topographiesystem und dem Computersystem zur Übertragung der Profildaten vom Topographiesystem zum Computersystem; und
einer zweiten Datenverbindung zwischen den Computersystem und der Laseraugenchirurgievorrichtung zur Übertragung des Anfangsabtragungsschußmusters und des verfeinerten Schußmusters aus dem Computersystem zur Laseraugenchirurgievorrichtung, wobei die Laseraugenchirurgievorrichtung in einer physikalisch anderen Umgebung als das Computersystem angeordnet ist.

9. Vorrichtung nach einem der Ansprüche 1 bis 8, wobei der Laser ein Excimerlaser ist.

10. Vorrichtung nach einem der Ansprüche 1 bis 9, wobei der Laserstrahl mit der größeren festen Fleckgröße einen Durchmesser von mindestens 2,0 mm aufweist.

11. Vorrichtung nach einem der Ansprüche 1 bis 10, wobei der Laserstrahl mit der kleineren festen Fleckgröße einen Durchmesser von nicht mehr als 1,0 mm aufweist.

12. Vorrichtung nach einem der Ansprüche 1 bis 11, wobei die Doppelfleckoptik aus einer Linse besteht.

13. Vorrichtung nach einem der Ansprüche 1 bis 12, wobei die Reihe von Schüssen mit der kleineren festen Fleckgröße aus überlappenden Schüssen besteht.

## Revendications

1. Appareil (10) pour façonner la cornée en éliminant du tissu dans une zone de la cornée qui présente une aire devant être soumise à ablation selon un schéma de traitement désiré, comprenant : un laser (20) qui émet un faisceau laser (22) ayant une longueur d'onde adaptée, un système optique (40, 42) couplé audit laser qui reçoit le faisceau laser et applique le faisceau laser sur la zone de la cornée, ledit système optique déviant de manière contrôlable le faisceau laser vers différents points sur la zone de la cornée,
**caractérisé par**
des optiques à point double (36, 38) couplées au système optique et restreignant le système optique pour fournir le faisceau laser à une seule taille de point fixe plus grande sur la zone de la cornée et à une taille de point fixe unique plus petite sur la zone de la cornée, la taille de point fixe plus grande sur la zone de la cornée étant une fraction relativement grande de la zone de la zone de la cornée devant être soumise à ablation, et la taille de point fixe plus petite sur la zone de la cornée étant relativement petite comparativement à la taille de point fixe plus grande ; et
un dispositif de commande (64) pour diriger le système optique et pour diriger ledit laser pour déclencher le faisceau laser et le système optique pour visualiser le faisceau laser en une série de tirs en une pluralité de points différents sur la zone de la cornée uniquement à la taille de point fixe plus grande et à la taille de point fixe plus petite, qui créent ensemble de manière cumulée le schéma de traitement désiré.

2. Appareil de la revendication 1, dans lequel l'optique à double point inclut un diagramme fixe double (36, 1000).

3. Appareil de la revendication 2, dans lequel le diaphragme fixe double est placé dans le trajet (22) du faisceau laser.

4. Appareil de la revendication 2, dans lequel le diaphragme fixe double glisse entre une première position et une seconde position.

5. Appareil de la revendication 2, dans lequel le diaphragme fixe double a une première ouverture (1002) et une seconde ouverture (1010) qui est plus petite que la première ouverture.

6. Appareil de la revendication 1, dans lequel l'optique à point double inclut un diaphragme fixe double placé dans le trajet du faisceau laser, le diaphragme fixe double glissant entre une première position et une seconde position et ayant une première ouverture et une seconde ouverture plus petite que la première ouverture, le faisceau laser traversant la première ouverture lorsque le diaphragme fixe double est dans le première position et la seconde ouverture lorsque le diaphragme fixe double est dans la seconde position.

7. Appareil de la revendication 1, dans lequel l'optique à point double est un diaphragme variable (36) qui est commandé de manière programmable pour prendre seulement deux tailles différentes.

8. Appareil selon l'une quelconque des revendications 1 à 7, **caractérisé en outre par**
un système de topographie (T1, T2, T3) qui fournit des données de profil correspondant au profil de la cornée d'un patient ;
un système informatisé (C1, C2) avec un programme pour développer une schéma de tir d'ablation initial et un schéma de tir affiné qui forment ensemble le schéma de tir désiré global à partir des données de profil ;
un premier lien de données entre ledit système de topographie et ledit système informatisé pour transmission des données de profil depuis ledit système de topographie vers ledit système informatisé ;
et
un second lien de données entre ledit système informatisé et ledit appareil de chirurgie laser de l'oeil pour transmission du schéma de tir d'ablation initial et du schéma de tir affiné depuis ledit système informatisé vers ledit appareil de chirurgie laser de l'oeil, dans lequel ledit appareil de chirurgie laser de l'oeil se trouve dans un lieu physiquement différent du système informatisé.

9. Appareil selon l'une quelconque des revendications 1 à 8, dans lequel le laser est un laser excimer.

10. Appareil selon l'une quelconque des revendications 1 à 9, dans lequel le faisceau laser de taille de point fixe plus grande a un diamètre d'au moins 2,0 mm.

11. Appareil selon l'une quelconque des revendications 1 à 10, dans lequel le faisceau laser de taille de point fixe plus petite a un diamètre ne dépassant pas 1,0 mm.

12. Appareil selon l'une quelconque des revendications 1 à 11, dans lequel l'optique de point double est une lentille.

13. Appareil selon l'une quelconque des revendications 1 à 12, dans lequel la série de tirs de taille de point fixe plus petite sont des tirs se chevauchant.
